# EUROPEAN PATENT APPLICATION

(11) **EP 3 967 231 A1**
(43) Date of publication of application: **16.03.2022**
(21) Application number: 20195505.1
(22) Date of filing: 10.09.2020
(51) Int. Cl.: A61B 6/00, G01N 23/041

(54) **MIXED-USE CONVENTIONAL AND GRATING-BASED IMAGING**

(71) Applicant: Koninklijke Philips N.V., 5656 AG Eindhoven (NL)
(72) Inventor: KOEHLER, Thomas, 5656 AE Eindhoven (NL)
(74) Representative: Philips Intellectual Property & Standards

(57) **Abstract**

The present invention relates to a grating-based X-ray imaging system (100), and a related grating assembly, method and computer-program product. The system comprises a radiation source (108) and a radiation image detector (110), in which the source and the detector are disposed at opposite sides of an examination region (106) such that, in use, radiation emitted by the source passes through the examination region and impinges on the detector. The system comprises an X-ray interferometer for dark-field and/or phase-contrast imaging, which comprises an analyzer grating (101) arranged on or proximate to the radiation image detector (110). The analyzer grating covers a fraction of the active area of the radiation image detector in the range of 10% to 90%, but does not cover the remainder (103) of the active area.

## Description

### FIELD OF THE INVENTION

The present invention relates to the field of X-ray dark-field and/or phase-contrast imaging, i.e. grating based X-ray imaging. More specifically, the invention relates to methods and systems for dark-field and/or phase-contrast imaging in an X-ray imaging system, e.g. a computed tomography (CT) system, that is adapted to support conventional X-ray attenuation imaging as well as grating-based imaging in a flexible manner, i.e. a mixed-use imaging system.

### BACKGROUND OF THE INVENTION

In conventional CT imaging, the obtained image contrast is representative of differences in X-ray radiation attenuation inside the scanned object. However, this imaging modality is less suitable in cases where different constituent materials with similar attenuation cross sections, e.g. normal and pathologic tissue in the body of a patient, need to be examined. Grating-based X-ray imaging techniques may offer a more suitable alternative in such cases. These techniques enable images to be constructed that are representative of phase contrast and/or dark-field contrast.

A grating-based imaging system uses a grating interferometer that comprises two or more gratings. A three-grating interferometer arrangement is often preferred, since it can be used, in principle, in combination with a conventional X-ray source and detector, e.g. as would be suitable for conventional attenuation X-ray imaging. The system may be adapted for differential phase contrast imaging, e.g. using a Talbot-Lau-type interferometer. For example, a grating-based imaging system may comprise a source grating, between the X-ray tube and the object to be imaged, while a phase grating and an analyzer grating are placed in between the object and the detector (spaced apart from each other in accordance with stringent distance requirements). The source grating creates an array of individually coherent, yet mutually incoherent, virtual sources, e.g. coherent beamlets (note however that alternative approaches to generate such coherent beamlets exist as well). The phase grating divides the beam into its first diffraction orders, while the analyzer grating acts as a transmission mask to transform local fringe variations into detectable intensity values. Variations exist that may rely on different types, a different number and/or different characteristics of the involved gratings, however grating-based phase-contrast and/or dark-field imaging techniques have the use of a plurality of phase and/or absorption gratings in common, which are positioned at well-defined positions in the beam path in accordance with positioning principles and rules. The specific configuration used for grating-based imaging, i.e. the positions, orientations and parameters of the components, needs to be accurately maintained during the imaging protocol and can be very sensitive to deviations.

X-ray dark-field imaging has the advantage that it is able to reveal information about structures at a scale below the limits imposed by the spatial resolution of the imaging system. For example, this imaging modality has been shown to have potential for improving the diagnostic accuracy of pulmonary disorders as compared to conventional X-ray attenuation imaging. This can be understood as a result of the high sensitivity of dark-field imaging to small-angle scattering of X-ray radiation by particularly small structures, for example by the alveoli (small structures in the lung that are responsible for gas exchange with the blood). However, the same technique may be useful for many other small structures of interest in the body, such as, for example, fine sponge-like structures in bone.

Notwithstanding a potential for significantly increasing diagnostic accuracy, e.g. of pulmonary disorders but possibly other conditions as well, the potential clinical value of dark-field imaging in CT has not yet been studied extensively. Therefore, a need exists to provide a dark-field CT imaging option in a conventional CT system at a low (additional) cost and without any substantial impediment to the use of that CT system in a conventional imaging mode, or at least such as to impose as little constraints on the conventional use of the system as possible. Another disadvantage of having only a limited number of studies relating to the clinical use of dark-field imaging is that it may be presently regarded as unclear what the resolution requirements for the dark-field image are (possibly depending on the intended diagnostic feature to be examined).

Likewise, X-ray phase-contrast imaging (also called phase-sensitive imaging) offers many advantages when compared to conventional attenuation imaging. In a sense, phase-contrast imaging, dark-field imaging and attenuation imaging are complementary imaging modalities, in which each modality can offer information about the imaged subject that is not revealed, or not revealed in the same level of detail, by the other modalities. For example, conventional X-ray attenuation imaging is particularly suitable for examining structures involving materials that cover a wide range of atomic number (Z). Phase-contrast imaging is sensitive to variations in the refractive index, which depends on the electron density. Soft tissues, which typically consist of materials having very similar atomic number (e.g. due to carbon, hydrogen and oxygen being the predominant constituent elements), can nonetheless have widely varying electron densities, e.g. due to different molecular compounds. Advantageously, these three, mutually complementary imaging modalities can be acquired in a single examination, e.g. by a single exposure (or at least in a single exposure sequence). Different processing algorithms can be applied to the same raw data to obtain, respectively, a differential phase-contrast image, a dark-field image and a conventional attenuation image.

A single image acquisition run can be used to concomitantly generate the three different (mutually complementary) types of image: an X-ray phase-contrast image, a dark-field image and a conventional attenuation image. Therefore, a conventional use of the system, in which only attenuation images are desired, is inherently supported by any dark-field imaging system. Unfortunately, by projecting the X-ray beam through several gratings, as used in a dark-field/phase-contrast image acquisition, a large fraction of the X-ray fluence generated at the source is lost in absorption (and/or scattering). This implies higher tube power (with higher operating and maintenance costs and/or decreased tube lifespan) as well as a rather large dose penalty to the imaged subject, e.g. roughly about a doubling of the received radiation dose, in order to achieve the same diagnostic image quality, e.g. such that the acquired attenuation image has the same contrast and noise level. The source grating strongly reduces the usable radiation fluence as function of the X-ray tube output, which is obviously disadvantageous, but does not directly affect the dose received by the imaged patient. However, the gratings placed between the imaged subject and the detector can have a strong effect on this dose (for the same image quality). This is particularly the case for the analyzer grating, which is typically an absorption grating.

It is clear that it is likely unacceptable to image a patient with a dark-field/phase-contrast acquisition protocol if only the attenuation image component is needed, in view of the dose penalty mentioned hereinabove, and particularly when the added clinical value of the dark-field/phase-contrast image is not yet firmly established without any doubt. As already mentioned, dark-field imaging is - at the current state of knowledge - mainly interesting for lung imaging, such that it is highly desirable to provide a smart approach for selectively enabling and disabling the dark-field (or phase-contrast) feature of a scanner. Instead of providing a dark-field imaging system with a fixed dose penalty of about a factor of two (e.g. caused by the attenuation of the analyzer grating), it may be preferable to design a system that allows a trade-off between the dose penalty and the dark-field image quality at constant conventional image quality, particularly since the dark-field image quality requirements for diagnostic use are still uncertain at the moment.

The analyzer grating used in grating-based imaging is a relatively large mechanical component that is placed in front of (e.g. close to) the imaging detector. To revert the imaging system to a conventional imaging mode, this analyzer grating can be moved out of the beam path.

However, this requires an actuator, or, otherwise, the manual removal of the grating from the beam path. Neither option is desirable. Automatic actuation adds to the complexity and the costs in manufacture and maintenance of the system. It is pointed out that this grating can be, typically, quite large, e.g. of a size similar to the detector, such that the size, complexity and cost of the required automation actuator would also be proportionate. Manual removal of the grid is difficult, or maybe even impossible, in view of the accurate alignment requirements for the grating components. Even if this would be feasible, it would still imply a loss of valuable time, a risk of potentially damaging the grating or detector, ergonomic issues, and/or might require additional staff training.

Furthermore, in order to remove the grating from the beam path, sufficient space is required around the slot or holder where the grating is installed. Preferably, the grating is moved to a secondary holder where it is held when not in use, e.g. such that it can be easily slid or swung back to its position for active use. However, this may require even more space to accommodate the grating when not in use, e.g. as a permanent part of the system.

It should be noted that this space issue is particularly troublesome in CT imaging, in which the detector is mounted on a moving gantry. Therefore, the analyzer grating, in use, needs to be supported by the gantry as well, which preferably would also apply to the grating when not in use (parked) to avoid a complex and costly mechanism to slide the grating out of a mobile part of the gantry into a stationary part of the system (taking precise alignment issues into account as well). Given the complexity of a typical CT system, usable space in and/or on the gantry, but also in the immediate vicinity of the gantry, may be very limited. For example, in addition to the gratings, other X-ray beam conditioning components, such as low energy filters, bow-tie filters and/or beam collimators, may need to be positioned between the X-ray tube and the detector as well, all while leaving enough room to accommodate a (potentially corpulent) patient to be imaged.

The volume and weight of moving parts of the system should be held as low as possible, for obvious mechanical reasons. The volume of the system may also need to be kept at a minimum to ensure that a subject can be easily and freely positioned inside the examination volume. Furthermore, the space around the system may need to be kept free or as uncluttered as possible, e.g. for aesthetic and hygienic reasons but possibly also to allow medical interventions to be carried out on the patient while imaging.

US 2019/336086 provides an example of an automated/actuated system to switch between a conventional CT imaging mode and a grating-based imaging mode. This document describes a grating-based X-ray imaging system, in which an interferometer grating support is used to support a source grating (G0), a phase or absorber grating (G1) and a bowtie filter. For a grating-based X-ray imaging scan, the grating support is moved into a region between a low energy photon filter and a beam collimator (i.e. at the source side of the examination region) and the third grating, the analyzer grating (G2), is moved into the beam path in a region between the examination region and the detector array (i.e. at the detector side of the examination region). In this approach, and possibly also in similar prior-art systems, the analyzer grating G2 may be actuated by a suitable electro-mechanical system, e.g. including a controller, a motor and/or a drive system.

### SUMMARY OF THE INVENTION

It is an object of embodiments of the present invention to provide good and efficient means and methods to allow the use of an imaging system for grating-based imaging as well as the use of the system in a conventional attenuation-only imaging mode.

It is an advantage of embodiments of the present invention that a dose penalty to an imaged patient due to radiation absorption in an analyzer grating can be reduced or avoided.

It is an advantage of embodiments of the present invention that a conventional attenuation-only image can be acquired by impinging radiation on only a part of the detector that is not covered by the analyzer grating.

It is an advantage of embodiments of the present invention that the analyzer grating does not require an actuator or slotting means to allow the (frequent) removal and reinsertion of the grating into the beam path. It is to be noted that the grating may still be removable and/or replaceable, e.g. for maintenance operations, but may typically remain fixed during operation of the device regardless of the imaging mode that is selected.

It is an advantage of embodiments of the present invention that a trade-off between the dose penalty to a patient and a dark-field and/or phase-contrast image quality can be configured, e.g. selected by manipulating controllable parameters in use of the system, for a constant image quality of a conventional attenuation image that is acquired simultaneously.

It is an advantage of embodiments of the present invention that a system is provided that can be easily operated, in which a conventional attenuation-only or a grating-based image can be acquired (and/or a combination of a phase-contrast/dark-field image and an attenuation image) as per a user selection, at a low cost of the system.

It is an advantage of methods and means in accordance with embodiments of the present invention that a cheap and/or efficient approach to provide grating-based image capabilities is provided without excessive constraints on the usability of the system for conventional attenuation-only imaging.

A system and method in accordance with embodiments of the present invention achieves the above objective.

In a first aspect, the present invention relates to a grating-based X-ray imaging system. The system comprises a radiation source and a radiation image detector, in which the radiation source and the radiation image detector are disposed at opposite sides of an examination region, such that, in use of the system, radiation emitted by the radiation source passes through the examination region and impinges on the radiation image detector. The system also comprises an X-ray interferometer for dark-field and/or phase-contrast imaging, which comprises an analyzer grating arranged on or proximate to the radiation image detector. The analyzer grating only covers a part of the active area of the radiation image detector, such as a fraction of the active area of the radiation image detector in the range of 10% to 90%, (e.g. 20% to 80%, e.g. 30% to 70%, e.g. 40% to 60%, e.g. 45% to 55%, e.g. 47% to 53%, e.g. about 50%, or any subrange formed by the mentioned points, such as 30% to 45%) but does not cover the remainder of the active area.

In an imaging system in accordance with embodiments of the present invention, the analyzer grating may be in a fixed (e.g. static) arrangement with respect to the radiation image detector. For example, the imaging system in accordance with at least some embodiments of the present invention does not comprise an actuator, e.g. a controllable and/or automated actuator, to move the analyzer grating with respect to the radiation image detector.

In an imaging system in accordance with embodiments of the present invention, the analyzer grating may cover one side of the detector from an edge of the detector over only a part (e.g. to about 10% to 90%) of the length of the detector along a first direction of the detector and may leave the remainder of the active area of the detector on the other side, opposite of said edge in said first direction, of the detector uncovered by the analyzer grating.

In an imaging system in accordance with embodiments of the present invention, the analyzer grating may cover both sides of the detector, at opposite ends of the detector in a first direction, while leaving a central part, in between said both opposite sides, uncovered.

In an imaging system in accordance with embodiments of the present invention, the analyzer grating may cover at least 90%, e.g. 95%, e.g. substantially 100%, e.g. 100%, of the detector in a direction perpendicular to said first direction, and this over the entire length of the detector in said first direction (i.e. for any path along the perpendicular direction through each position along the first direction).

The first direction (as well as the direction perpendicular to this first direction) may specifically refer to a principal direction in which the (possibly curved) plane of the detector extends, e.g. does not refer to a thickness dimension. For a curved detector, these directions may instead refer to paths following the curvature of the detector, even though, in the strictest sense, the direction would change along such path.

In an imaging system in accordance with embodiments of the present invention, the x-ray interferometer may comprise a source grating and/or a third (e.g. phase) grating. The system may further comprise an actuator mechanism for controllably moving the source grating and/or the third grating into and out of the beam path of radiation emitted by the radiation source to impinge on the radiation detector.

An imaging system in accordance with embodiments of the present invention may comprise an adjustable collimator to block part of the radiation emitted by the radiation source such as to illuminate a selectable part of the detector by the unblocked portion of the beam emitted by the source.

An imaging system in accordance with embodiments of the present invention may comprise a controller for controlling the adjustable collimator such as to select from different supported modes of operation, in which said different modes differ in the ratio of the detector area exposed to radiation emitted by the radiation source and covered by the analyzer grating and the detector area exposed to radiation emitted by the radiation source and uncovered by the analyzer grating.

An imaging system in accordance with embodiments of the present invention may comprise a rotatable gantry and/or an actuatable arm (or arms) onto which the radiation source and the radiation image detector are mounted at opposite positions with respect to each other across the examination region such that the radiation source and the radiation image detector can be moved in a coordinated motion to acquire projection images of the examination region from a plurality of different projection directions.

An imaging system in accordance with embodiments of the present invention may be a computed tomography system, in which the imaging system may comprise a stationary gantry supporting the rotatable gantry. The rotatable gantry may be adapted for rotating around the examination region around a longitudinal axis.

An imaging system in accordance with embodiments of the present invention may comprise a reconstructor for reconstructing tomographic images from signals provided by the image detector, in which the reconstructor may be adapted for performing a phase contrast and/or dark-field image reconstruction based on those of the signals that were obtained by elements of the image detector covered by the analyzer grating and that were exposed to radiation emitted by the radiation source, and for performing an attenuation image reconstruction based on all of the signals that were obtained by elements of the image detector that were exposed to radiation emitted by the radiation source, e.g. regardless of passing through the analyzer grating or not.

An imaging system in accordance with embodiments of the present invention may comprise a support, e.g. a couch, for supporting an object or subject to be imaged in the examination region, in which the support comprises one or more actuators to move the support, and the subject or object thereon, through the examination region such as to scan the subject or object over a length in the longitudinal direction.

In an imaging system in accordance with embodiments of the present invention, the analyzer grating may be positioned and/or oriented such that it entirely covers the angular arc that is subtended by the radiation detector, yet not the entire width of the radiation detector in the longitudinal direction.

In a second aspect, the present invention relates to an interferometer grating assembly comprising a mounting frame encompassing an area, in which the analyzer grating covers a fraction of this area in the range of 10% to 90%, and in which the remainder of the area is left open or filled with a material that does not significantly attenuate X-ray radiation.

In a third aspect, the present invention relates to a method, e.g. a computer-implemented method, for operating a grating-based X-ray imaging system that comprises a radiation source and a radiation image detector, disposed at opposite sides of an examination region, and an X-ray interferometer for dark-field and/or phase-contrast imaging. The interferometer comprises an analyzer grating arranged on or proximate to the radiation image detector, which only covers a part of the active area of the radiation image detector, such as a fraction in the range of 10% to 90% (or any of the alternative ranges mentioned hereinabove), but does not cover the remainder of the active area

The method comprises controlling a collimator of the system to collimate a radiation beam emitted by the radiation source such as to configure a (selectable) ratio of the detector area exposed to the radiation beam for which the radiation passes through the analyzer grating relative to the detector area exposed to the radiation beam for which the radiation does not pass through the analyzer grating,

The method may comprise, determining the fraction based on a user input, e.g. received via a user interface.

The method comprises emitting (or controlling the radiation source to emit) said radiation beam by the radiation source and through the examination region to impinge on the radiation image detector. The method may comprise obtaining signals from the radiation image detector indicative of the radiation impinging thereon.

The method comprises forming a first image representative of radiation attenuation of an object or subject placed in the examination area by processing image signals provided by all of the detector elements of said radiation image detector that were exposed to the radiation beam, and, if said (selected) ratio is non-zero, forming at least a second image representative of phase contrast and/or small-angle scattering by processing image signals provided by only the detector elements that were exposed to the radiation beam where it passed through the analyzer grating.

In a fourth aspect, the present invention relates to a computer-program product for performing a method in accordance with embodiments of the third aspect of the present invention when executed on a computer.

The independent and dependent claims describe specific and preferred features of the invention. Features of the dependent claims can be combined with features of the independent claims and with features of other dependent claims as deemed appropriate, and not necessarily only as explicitly stated in the claims.

### BRIEF DESCRIPTION OF THE DRAWINGS

Fig. 1 shows a system in accordance with embodiments of the present invention.
Fig. 2 illustrates further configurable modes of operation of a system in accordance with embodiments of the present invention.
Fig. 3 shows another illustrative system in accordance with embodiments of the present invention.
Fig. 4 shows an interferometer grating assembly in accordance with embodiments of the present invention.
Fig. 5 shows a computed tomography system in accordance with embodiments of the present invention.
Fig. 6 illustrates a method in accordance with embodiments of the present invention.

The drawings are schematic and not limiting. Elements in the drawings are not necessarily represented on scale. The present invention is not necessarily limited to the specific embodiments of the present invention as shown in the drawings.

### DETAILED DESCRIPTION OF EMBODIMENTS

Notwithstanding the exemplary embodiments described hereinbelow, is the present invention only limited by the attached claims. The attached claims are hereby explicitly incorporated in this detailed description, in which each claim, and each combination of claims as allowed for by the dependency structure defined by the claims, forms a separate embodiment of the present invention.

The word "comprise," as used in the claims, is not limited to the features, elements or steps as described thereafter, and does not exclude additional features, elements or steps. This therefore specifies the presence of the mentioned features without excluding a further presence or addition of one or more features.

In this detailed description, various specific details are presented. Embodiments of the present invention can be carried out without these specific details. Furthermore, well-known features, elements and/or steps are not necessarily described in detail for the sake of clarity and conciseness of the present disclosure.

In a first aspect, the present invention relates to a grating-based X-ray imaging system.

The grating-based X-ray imaging system is adapted for acquiring images in substantially a conventional X-ray attenuation image acquisition mode, but also in a grating-based image acquisition mode, such as a phase-contrast and/or dark-field imaging mode and/or a mixed acquisition mode. Each mode can be selected and activated separately, i.e. a user can select in which mode the system is to operate for a specific image acquisition. The selectable modes can be discrete, e.g. a selection between a conventional attenuation imaging mode and a grating-based imaging mode (and/or a mixed acquisition mode), and/or can be continuously or in a plurality of fine steps configured to select a desired ratio of direct attenuation image acquisition and grating-based image acquisition.

The grating-based X-ray imaging system may be an actuated system for dynamically acquiring projection images from a plurality of different viewpoints, such that an object can be viewed from different angles and/or projection directions. As is well-known in the art, such different projection views can be combined into a derived image that shows a larger field of view than acquired in a single acquisition and/or in a tomographic (or tomosynthetic) reconstruction that shows the internal composition of the imaged object in a plane (or across a volume) that does not correspond to the direction of (any of the) projection(s), e.g. a cross-section (without limitation of the plane of reconstruction to necessarily perpendicular cross-sections). Thus, even though only part of the detector is provided with an analyzer grating, as will be explained hereinbelow, the entire object to be imaged (or a desired part thereof) can be studied by combining information from a plurality of projection images from different views.

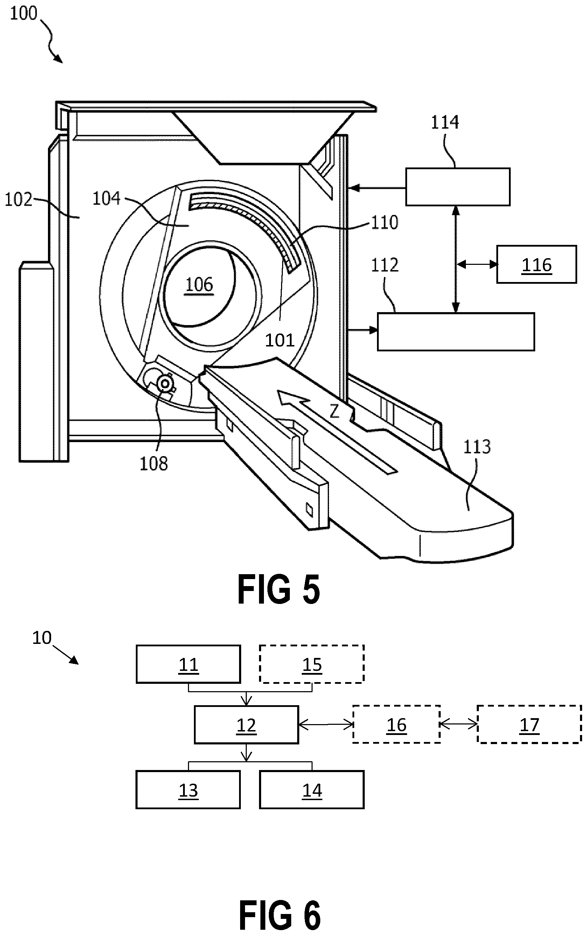

The grating-based X-ray imaging system may be a computed tomography (CT) system, particularly one such system that is suitable for acquiring images in substantially a conventional X-ray attenuation image acquisition mode, but also in a grating-based image acquisition mode, such as a phase-contrast and/or dark-field imaging mode (and/or a combination of grating-based image acquisition and attenuation image acquisition). A CT system in accordance with embodiments of the present invention is schematically illustrated in Fig. 5.

However, embodiments are not necessarily limited to CT systems. The imaging system may be a conventional projection imaging system, e.g. a direct radiography system or a mammography system. Nonetheless, it is noted that embodiments of the present invention may be particularly advantageous when applied to CT systems, since mechanical components for dynamically moving an analyzer grating into and out of the beam path can be avoided in accordance with embodiments of the present invention. Such mechanical components, e.g. guiding rails and/or actuators, can be heavy and bulky, and therefore particularly disadvantageous in view of the added weight and/or volume when installed on a CT gantry. The same reasoning may apply to any system in which an imaging protocol involves automated and coordinated movement of the image detector during the image acquisition (or at least in between a sequence of coordinated image acquisitions). Other examples of such systems may include tomosynthesis systems, C-arm imaging systems, and/or interventional radiology systems (e.g. catheter guidance suites), without any limitation of embodiments thereto. Nonetheless, principles of the present invention may also be applied to a system that is primarily intended for capturing one or only a few static projection images per examination, even though the advantages of applying the present invention to such system might be less pronounced (yet still tangible). Furthermore, the flexibility of an actuated system for dynamically acquiring projection images from a plurality of different viewpoints, e.g. a CT system, a C-arm system or the like, can advantageously compensate for a loss of usable detector area in accordance with a selected imaging mode as discussed further hereinbelow.

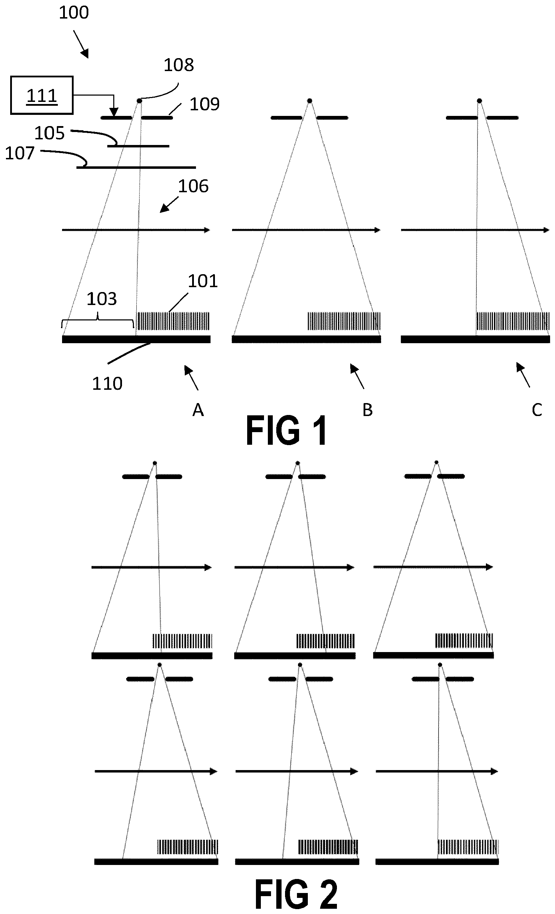

Referring to Fig. 1, a system 100 in accordance with embodiments of the present invention comprises a radiation source 108, and a radiation image detector 110, disposed at opposite sides of an examination region 106.

The system may comprise a rotatable gantry 104 (see e.g. Fig. 5) or actuated arm (i.e. adapted for rotating in operation of the system or moving in at least one degree of freedom of rotation and/or translation). The radiation source 108 and the radiation image detector 110 may be held by the gantry or arm at opposite positions with respect to each other across the examination region 106, such that the radiation source 108 can project X-ray radiation through the examination region 106 to impinge on the radiation image detector, for example in a CT system as shown in Fig. 5.

The system comprises an X-ray interferometer for use in dark-field and/or phase-contrast imaging, the interferometer comprising at least one analyzer grating 101 arranged on or near the radiation image detector, e.g. between the examination region 106 and the image detector 110, typically at a relatively short distance to the detector.

The analyzer grating may comprise a pattern of alternating lines of higher and lower (or substantially zero) absorption for the radiation quality emitted by the source. Such analyzer gratings are known in the art and not discussed in detail. The analyzer grating is not necessarily limited to a line pattern, and may comprise any suitable pattern of adjacent high attenuation and low attenuation regions with a sufficiently high spatial frequency to analyze the phase and/or small angle scattering information into a pattern of radiation intensity contrast that is discernable by the pixelated detector. The grating may be flat, but is not necessarily so. For example, the grating may be curved, e.g. to follow a curve of the detector (for example as commonly used in CT systems).

The analyzer grating 101 only covers a part of the active area of the radiation image detector, such as a fraction of the detector in the range of 10% to 90%, preferably in the range of 20% to 80%, e.g. in the range of 30% to 70%, e.g. in the range of 40% to 60%, e.g. in the range of 45% to 55%, e.g. in the range of 47% to 53%, e.g. in the range of 48% to 52%. For example, the analyzer grating may cover about 50% of the detector (embodiments not necessarily limited thereto). "Covering" refers to the projection of the analyzer grating onto the detector when considering the radiation source (e.g. the focal spot of the radiation source) as source of a point projection, e.g. to the area of a shadow cast from the source by the grating onto the detector. Thus, the fraction refers to the area of the projected umbra relative to the active area of the detector, i.e. the active area over which the image detector is adapted for transducing radiation into signals representative of a formed image. Since a grating, particularly an absorption grating, typically comprises grating lines or a grating pattern that provides an alternating pattern of higher and lower (or no) attenuation of radiation, it shall be understood that the fraction includes both the area covered by the high attenuation lines (or other pattern structure) and by the low (or substantially zero) attenuation lines that together form the grating pattern, but does not account for large-scale openings where the grating is not present, i.e. the remainder of the fraction in the range of 10% to 90%. Therefore, the analyzer grating covers a fraction of the detector in the range of 10% to 90% (possibly a smaller range as stated hereinabove), but does not cover the remainder 103 of the active area of the detector.

As shown in Fig. 1, the analyzer grating 101 may cover one side of the detector, e.g. from an edge of the detector over only a part, such as to about 10% to 90%, e.g. 20% to 80%, e.g. 30% to 70%, e.g. 40% to 60%, e.g. about half, along the length of the detector. The analyzer grating may cover, in a direction perpendicular to this lengthwise direction at least 80%, e.g. at least 90%, e.g. 95%, e.g. about 100% of the width of the detector. If the distance of the analyzer grating to the detector is not negligible, even though this may be the case for many embodiments of the present invention, these ranges refer to the dimensions of a shadow cast by the analyzer grating onto the plane of the detector when considering a point projection from a focal spot of the source. The grating lines of the analyzer grating may extend in the lengthwise direction or in the widthwise direction, without necessarily being limited to such aligned orientations.

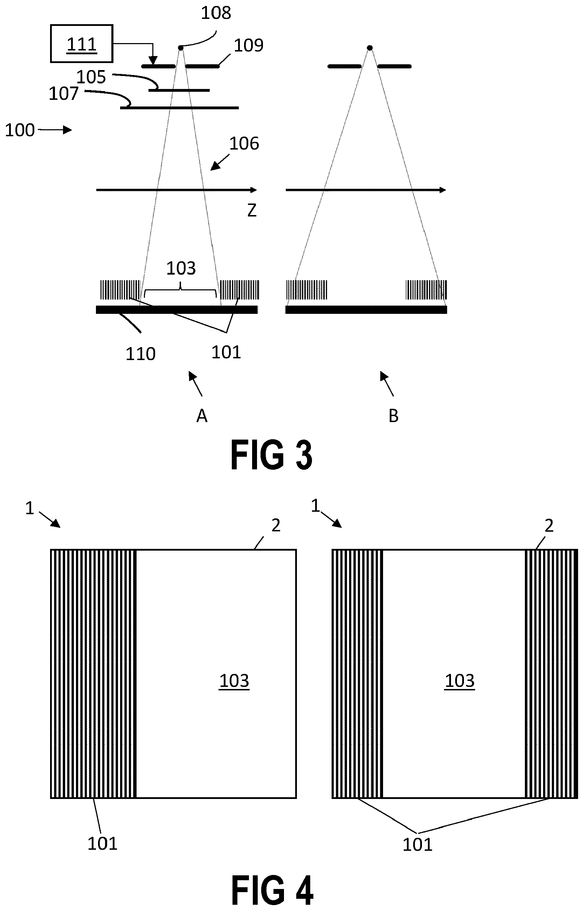

However, embodiments of the present invention are not limited to such a two-side partitioning of the detector in a side that is covered by the analyzer grating and another side that is not. For example, as shown in Fig. 3, the analyzer grating may cover both sides of the detector, while leaving a central part 103 uncovered. It is to be noted that the analyzer grating may consist of two (or more) separate (disjoint) grating elements, e.g. is not to be interpreted as necessarily a single contiguous grating. Nonetheless, the analyzer grating may comprise a mounting fixture or frame to hold such separate grating elements in a fixed position relative to each other. Even if the analyzer grating consists of a single contiguous grating element (see e.g. Fig. 1), a frame or mounting fixture may still be provided that may be dimensioned to correspond substantially with the dimensions of the detector, e.g. having a window or opening in the area encompassed by the frame or mounting fixture, for example for ease of alignment and positioning relative to the detector. For example, it may be advantageously easy to upgrade a system designed to hold a fixed, full-size analyzer grating by installing an analyzer grating in accordance with embodiments if the analyzer grating comprises a frame that is dimensioned commensurate with a full-size analyzer grating, even though the actual grating elements cover substantially less than area encompassed by the frame.

Thus, the grating may cover the outer detector rows while leaving the inner detector rows uncovered. A same ratio as discussed hereinabove of a covered part (consisting of two non-contiguous regions on both sides) relative to the total active area of the detector may apply. It will be appreciated that such configuration may be advantageous, since by collimating the beam to the central uncovered part, the behavior of the imaging system is substantially identical to the behavior of a conventional system (without phase-contrast/dark-field imaging capabilities), albeit with a smaller detector area. Particularly, a symmetric projection through the imaged subject is obtained in contrast to a slightly skewed view compared to the system illustrated in Fig. 1 (view A). Furthermore, where collimation of the beam in the case of a system as illustrated in Fig. 1 may require two separately controllable settings of the collimator, for each side of the collimation window, the system of Fig. 3 may suffice with one degree of controllable freedom of the collimator, e.g. opening and closing both sides of the collimation window in a symmetric manner, e.g. as illustrated by the configurations A and B in Fig. 3.

Even though the specific configuration of the interferometer may not be essential to the present invention, and different alternative configurations are known in the art, the interferometer may comprise, for example, a source grating 105, different from the analyzer grating 101, and a third grating 107, different from the source grating and different from the analyzer grating. The source grating may, typically, be disposed near the radiation source, i.e. between the radiation source and the examination region. Even though not commonly used, the system may use a different approach, instead of a source grating, to generate a coherent beam or locally coherent beamlets, e.g. a monochromatic source or a different type of monochromator to condition the radiation emitted by a polychromatic source.

The interferometer may be adapted for Shearing interferometry, or X-ray Talbot interferometry, e.g. a Talbot-Lau interferometer. The system may be adapted for Electronic Phase Stepping (but embodiments are not limited thereto). The interferometer may be adapted for an alternative or variant of X-ray interferometry as known in the art, e.g. Edge-illumination (embodiments not limited thereto). In the latter example, it will be understood that the analyzer grating may specifically refer to an aperture mask on the detector (that partially covers the active area by an aperture pattern), which is at least similar to a grating but performs the phase analyzer function in a slightly different manner (i.e. using the effect of detector element edge illumination). It will also be understood that, certainly for embodiments where the analyzer grating refers to an aperture coding mask (but not necessarily only for such embodiments), embodiments are not limited to line "grating" patterns, e.g. may also refer to L-shaped apertures or analogues thereof.

The third grating 107 may be a phase grating (or absorber grating, for some variants of grating-based imaging techniques), the position of which may vary across embodiments (e.g. including embodiments where the third grating is positioned in between the examination region and the source as well as embodiments where the third grating is positioned in between the examination region and the detector) but may be typically well-defined in accordance with an interferometric phase and/or small-angle scattering information encoding approach as known in the art. For example, this third grating may be positioned such as to use the self-imaging phenomenon, or Talbot effect, to encode phase and/or small-angle scattering information in the (typically intensity-dependent) signals detected by the detector, in combination with the analyzer grating. Even though different naming conventions can also be used in the art, the source grating will be referred to as the G0 grating, the third grating 107 as the G1 grating, and the analyzer grating 101 as the G2 grating.

While a dose penalty of using a grating-based imaging technique, relative to a conventional attenuation imaging technique (for a same or similar image quality), is mostly due to absorption at the analyzer grating, it may still be preferable (but not strictly necessary) to include a mechanism to remove the source grating 105, G0, from the beam path when only an attenuation image is desired. Without limitation thereto, reference is made to US 2019/336086, which discloses a suitable interferometer grating support for removing the phase grating from the beam path when not in use. Thus, the system in accordance with embodiments of the present invention may comprise an actuator mechanism to move the source grating (and possibly also the third grating G1) into and out of the beam path, e.g. as disclosed in the document referred to or an alternative for this as known in the art. While the impact on radiation dose may not be an essential consideration, it is still advantageous to provide means to remove the source grating from the beam path to reduce the required output requirements of the radiation source, e.g. to avoid wasting power and/or to extend life of the source and/or to reduce the radiation-induced aging of the gratings.

The system may comprise an adjustable collimator 109, preferably an automatically adjustable collimator, e.g. driven by an actuator and controlled by a controller, to block part of the radiation emitted by the source such as to illuminate a selectable part of the detector by the (unblocked part of the) beam emitted by the source. As shown in the different modes A,B,C in Fig. 1, and modes A and B in Fig. 3, this allows to select different modes of operation, in which either only the part 103 of the detector that is not covered by the analyzer grating 101 is used (view A), the entire detector is used (view B) or only the part of the detector that is covered by the analyzer grating 101 is used (view C). It is however to be noted that the system of Fig. 1 may offer a higher degree of configurability, e.g. the third mode C in which only the part of the detector that is covered by the analyzer grating is actively used may be unsupported in a system as shown in Fig. 3, even though this might be overcome by a more complex collimator design.

It will be understood that intermediate configurations are not necessarily excluded, e.g. in which both a fraction f1 (0 < f1 < 1) of the analyzer grating and a fraction f2 (0 < f2 < 1) of the uncovered area 103 are illuminated. Such versatility in configuring the system may allow, advantageously, to (e.g. continuously, or at least in a plurality of intermediate steps) select a trade-off between a dose-penalty (due to part of the beam being transmitted through the analyzer grating) and image quality of a conventional attenuation image and phase-contrast and/or dark-field images. For example, Fig. 2 illustrates different intermediate configurations of the collimator to balance the image quality of the attenuation and the dark-field/phase-contrast images for a fixed patient dose.

The system may comprise a controller 111 to control the adjustable collimator to select a part of the detector to be illuminated by the source and to block radiation from the source from impinging on the remainder of the detector. Thus, a conventional image may be acquired by blocking radiation from passing through the analyzer grating and a grating-based (e.g. phase-contrast and/or dark-field) image can be acquired by allowing radiation to pass through the analyzer grating. The controller may also be adapted to select a mixed-mode operation in which radiation passes through both the analyzer grating and via the uncovered part of the detector. In embodiments of the present invention, the controller may be adapted to select a configurable (less than whole) part of the analyzer grating to pass radiation therethrough and/or a configurable (less than whole) part of the region of the detector that is not covered by the analyzer grating. In embodiments of the present invention, the controller may also be adapted to block radiation from impinging on the uncovered part of the detector (embodiments not necessarily limited thereto).

The skilled person would also realize that an equivalent approach can be achieved by allowing the position of the source to vary with respect to a fixed collimator, even though this may be less practical. Thus, the adjustable collimator may equally comprise a fixed collimator and an actuator to adjust the position of the source, or a combination of a movable source spot and an automated collimator for even higher flexibility. Nonetheless, since phase-contrast and/or dark-field imaging may rely on precise spacing distances of the various components of the system, actuating the source with respect to a fixed or less configurable collimator may complicate the design of the system, even though a fixed and well-defined position of the source for a grating-based imaging mode is still compatible with the more flexible positioning requirements of the source for conventional attenuation imaging. Furthermore, it is also noted that embodiments may relate to a system that implements an electronic phase stepping (EPS) technique, in which already some degree of movement of the focal spot of the source may need to be provided.

The controller 111, or a further controller, may also be adapted for controlling an actuator mechanism for moving a source grating and/or a third grating into and out of the beam path.

Bearing in mind that, as mentioned hereinabove, principles of the present invention may equally be applied to different types of X-ray imaging system, an illustrative grating-based X-ray imaging system 100 in accordance with embodiments of the present invention, in the form of a computed tomography system, is shown in Fig. 3. Thus, the imaging system may comprise a computed tomography (CT) scanner.

The imaging system 100 may comprise a generally stationary gantry 102 and a rotating (rotatable) gantry 104. The rotating gantry 104 may be rotatably supported by the stationary gantry 102 and may (in use of the system) rotate around an examination region 106 about a longitudinal axis Z.

A radiation source 108, such as an x-ray tube, may be rotatably supported by the rotating gantry 104, e.g. such as to rotate with this rotating gantry 104, and may be adapted for emitting X-ray radiation that traverses the examination region 106. The radiation source 108 may comprise, or consist of, at least one x-ray tube.

The radiation image detector 110 may comprise a radiation sensitive detector array that subtends an angular arc opposite the radiation source 108 across the examination region 106. The image detector 110 may include a plurality of rows of detector elements arranged with respect to each other along the Z-axis direction. The image detector 110 may be adapted for detecting radiation traversing the examination region 106, and generating signals indicative thereof, e.g. indicative of the intensity of received radiation as function of the location of detection on the array.

The image detector may be a conventional CT image detector, but may also be more complex, e.g. may be able to distinguish incident radiation as dependent on its energy, e.g. a dual-energy detector or spectral detector. The radiation image detector may, for example, comprise one or more scintillators and a plurality of photosensors, a direct conversion detector or another type of radiation image detector as known in the art.

The CT system, as is known in the art, may comprise a reconstructor 112 for reconstructing the signals provided by the image detector 110. The reconstructor may be adapted to generate reconstructed tomographic images, e.g. showing transverse slices. It will also be understood that the CT system may merely acquire raw projection image data that can be used by an external workstation for generating reconstructed images, i.e. the CT system does not necessarily comprise the reconstructor.

The system may comprise a subject support 113, such as a couch, for supporting an object or subject in the examination region. The subject support 113 may comprise one or more actuators to move the support, and the subject supported thereon, to a selectable and/or controllable position, e.g. such as to move a selectable part of the subject into the examination region. Furthermore, the actuator or actuators may be adapted for moving, continuously or in steps, the subject through the examination region such as to scan (i.e. image) the subject over a length in the longitudinal direction Z that is greater than the length of the examination region as such, i.e. greater than the length of the detector (as determined by the number of detector rows and element size).

Furthermore, the analyzer grating 101 may be oriented such that it covers (substantially) the full arc covered by the detector, yet not the entire width in the longitudinal direction Z over which the imaged subject is translated during a scan operation, e.g. the direction Z perpendicular to the curvature of said arc. Thus, by limiting the beam to only the part of the detector that is covered by the grating, or to only the part of the detector that is left uncovered by the grating, the full field of view of the detector remains available for imaging in respectively a purely grating-based imaging mode or a purely attenuation based imaging mode, even though less than the total number of detector rows can be used. In other words, the CT system behaves as an equivalent CT system with fewer detector rows. This affects the scan pitch that needs to be selected to obtain the same level of image quality, e.g. the pitch would correspond to a value that would be selected for the equivalent system having fewer detector rows instead of a CT system with the same number of rows but without the partial coverage of the detector with the analyzer grating in accordance with embodiments of the present invention. However, the reconstruction performed by the reconstructor does not require extensive changes, i.e. can apply its processing as if the system were a CT scanner with fewer detector rows.

Attention is drawn to the example discussed hereinabove, relating to Fig. 3, in which a purely grating-based image mode (i.e. in which only the part of the detector is exposed to the beam that is covered by the analyzer grating) may not be available across all possible embodiments (at least without implementing a more complex collimator that blocks only a central part of the beam, which is not necessarily excluded). Nonetheless, a scan can still be performed in either a purely attenuation based imaging mode and a mixed mode, where at least some phase contrast or small-angle scattering information is collected in addition to attenuation based image information, and possibly for different (configurable) levels of image quality of the additional phase-contrast/dark-field image information by exposing a configurable fraction of the beam to the analyzer grating.

The reconstructor may also be adapted to adjust a gain (or perform other suitable preprocessing) of the signals obtained by the detector elements that are in use not covered by the analyzer grating than for those elements that are covered by the grating, such as to compensate for the additional attenuation due to the analyzer grating. By applying such differential approach, mixed-mode imaging can be obtained, i.e. in which, in a single scan, both signals are acquired from detector elements that are left uncovered by the analyzer grating and from detector elements that are covered by the analyzer grating, to reconstruct a conventional attenuation based image. Likewise, only the signals obtained by detector elements for which the analyzer grating is positioned in the beam path may be used to reconstruct a dark-field and/or phase-contrast image. By adjusting the collimation, a trade-off can be selected (for at least some embodiments that allow a plurality of intermediate collimation steps or a continuous selection of collimation) between image quality of the dark-field and/or phase contrast image and the conventional attenuation image, for a given dose level to the patient.

The analyzer grating 101 may also be oriented such that it covers only a part of the full arc subtended by the detector, and leaving another part of the full arc uncovered. Thus, for each detector row, a first part of the detector row may be covered by the analyzer grating while another part of the same row is not. While this might require more involved processing to reconstruct the image, and while this might lead to inhomogeneous image quality (for example resulting in a lower signal to noise ratio in peripheral parts of the reconstructed attenuation transverse image than in the central part, e.g. for a configuration as shown in Fig. 3) this may be acceptable depending on the intended application.

Typical reconstruction algorithms used in the art may easily be configured or adapted to handle such different behavior of individual detector signals with respect to each other. Furthermore, for a collimation as substantially illustrated in Fig. 1, which may create an asymmetric beam shape, reconstruction algorithms as known in the art, for example an aperture weighted wedge reconstruction algorithm, can easily compensate for (or be adapted to compensate for) the asymmetric shape of the part of the detector that is actively used (i.e. not excluded by the collimation) in a selected imaging mode. Even though this may create a slight degradation in image quality, e.g. for axial acquisitions, this can be considered as acceptable in view of the benefits of the added flexibility of the system, or can nonetheless be overcome by opting for a more symmetric approach for collimation, e.g. as illustrated in Fig. 3.

The system may also comprise an operator console 114, e.g. a general-purpose computer that is specifically programmed and/or configured for controlling or monitoring the system 100 and/or for providing a user interface to an operator. The console 114 may include a human readable output device such as a monitor or display and an input device such as a keyboard and mouse. Software resident on the console 114 may allow the operator to interact with the scanner 100 via a graphical user interface (GUI) or otherwise. This interaction may include selecting an imaging protocol, parameters of the system to apply in scanning a subject, a mode of operation, where different modes differ in the ratio of detector area exposed to radiation from the source and passing through the analyzer grating and detector area exposed to radiation from the source and not passing through the analyzer grating, e.g. by controlling the collimator, and/or other configurable settings as known in the art for such systems.

The imaging system 100 may be operably connected to a workstation, e.g. computing system 116, such as a computer, that may comprise an input/output (I/O) interface for facilitating communication with the CT scanner. The computing system 116 may comprise an output device. The output device or output devices may comprise, for example, a display monitor, a film printer, a paper printer and/or an audio output for audio feedback. The computing system may also comprise an input device or input devices, such as a mouse, a keyboard, a touch interface and/or a voice recognition interface. The computing system may also comprise at least one processor, such as a central processing unit (CPU), a microprocessor, a dedicated application-specific integrated circuit (ASIC) for processing and/or an appropriately configured programmable hardware processor such as a field-programmable gate array. The computing system may comprise a computer readable storage medium, e.g. a non-transitory memory such as a physical digital memory. The computer readable storage medium may store computer readable instructions and data. The at least one processor may be adapted for executing the computer readable instructions. The at least one processor may also execute computer readable instructions carried by a signal, carrier wave or other transitory medium. Alternatively or additionally, the at least one processor may be physically configured to embody the instructions, e.g. entirely or in part, without necessarily requiring memory storage of these instructions, e.g. by configuration of a field-programmable gate array or an ASIC specifically designed to carry out at least a part of the instructions.

The computing system may be comprised in the imaging system or may be external to it. The reconstructor 112 may be integrated in the computing system or may be embodied in a separate computing and/or processing device. The console 114 may be integrated in the computing system or may be embodied in a separate computing and/or processing device.

The console 114 may comprise the controller for controlling the collimator 109 or may be operably connected thereto. Thus, a user may be provided with an option, via the console, to select the type of scan to perform from a conventional imaging mode and a mixed-mode and/or grating-based imaging mode, and/or to select a weight that defines a priority assigned to image quality of reconstructed phase-contrast and/or dark-field images relative to image quality of attenuation based images, possibly including a target dose to the patient as constraint. Thus, a conventional CT (and/or scout scan) acquisition can be performed by collimation to the detector area not covered by the analyzer grating, while, if needed, dark-field and/or phase-contrast images can be simultaneously collected by including (or limiting to) at least part of the area covered by the grating.

Dark-field and/or phase-contrast CT acquisition can be performed by illuminating the entire detector (partially through the analyzer grating and partially not therethrough), or by illuminating only the part covered by the analyzer grating G2. While the same table speed limitations apply to both modes (e.g. a single selected pitch applies to the entire scan), they differ in the quality of the attenuation image that is acquired simultaneously. Acquisition with a full collimation will provide a better conventional image at the same patient dose than the acquisition with a smaller collimation, and the quality can thus potentially be finetuned to a desired level by selecting more or less coverage of the uncovered detector part.

In a second aspect, the present invention relates to an interferometer grating assembly 1, e.g. as illustrated in Fig. 4, that comprises a mounting frame 2 encompassing an area, which is commensurate with the dimensions of a radiation image detector onto which the interferometer grating assembly is intended to be mounted, and an analyzer grating 101 held in said frame, wherein said analyzer grating covers a fraction of said area in the range of 10% to 90%, or in the range of 20% to 80%, or in the range of 30% to 70%, e.g. in the range of 40% to 60%, e.g. in the range of 45% to 55%, e.g. about 50%, and wherein a remainder 103 of said fraction of the area is left open or filled with a material that does not significantly attenuate X-ray radiation, e.g. a low atomic number material, such as a thin sheet of a polymer, e.g. having a thickness of less than 0.5 cm. As discussed hereinabove, the grating may be a single contiguous grating on one side of the area (e.g. up to the edge of the frame), or may comprise a plurality of disjunct grating parts, such as covering two opposite sides of the area while leaving the center open (or filled with a substantially X-ray transparent material). As known in the art, the analyzer grating may be an absorption grating. The analyzer grating may comprise a plurality of grating lines. The frame and/or the analyzer grating may be flat (planar) or may be curved in at least one direction, e.g. such as to follow an arc of a curved detector of a CT scanner.

In a third aspect, the present invention relates to a method for operating a grating-based X-ray imaging system that comprises a radiation source and a radiation image detector, disposed at opposite sides of an examination region, and an X-ray interferometer for dark-field and/or phase-contrast imaging. The interferometer comprises an analyzer grating arranged on or proximate to the radiation image detector, in which the analyzer grating only covers a part of the active area of the radiation image detector, such as a fraction in the range of 10% to 90%, but does not cover the remainder of the active area. The grating-based imaging system may be a system in accordance with the first aspect of the present invention. Features of an imaging system in accordance with embodiments of the first aspect of the present invention may also apply, (if needed) mutatis mutandis, to a method in accordance with embodiments of the present invention.

Fig. 6 shows an illustrative method 10 in accordance with embodiments of the present invention.

The method comprises controlling 11 a collimator of the system for collimating a radiation beam emitted by the radiation source such as to configure a ratio of detector area exposed to the radiation beam for which the radiation passes through the analyzer grating relative to detector area exposed to the radiation beam for which the radiation does not pass through the analyzer grating.

The method comprises emitting 12 said radiation beam by the radiation source and through the examination region to impinge on the radiation image detector.

Emitting the radiation beam may comprise letting the radiation beam pass through a source grating of the interferometer and/or a third grating, e.g. a phase grating, of the interferometer before passing through the analyzer grating.

The method may also comprise controlling 15 an actuator to move the source grating and/or the third grating into and out of the path of the radiation beam. Particularly, if said configured ratio is zero, i.e. the detector is not exposed to radiation passing through the analyzer grating, the source grating and/or third grating may be moved out of the beam path, while for a non-zero ratio, the source grating and/or the third grating may be moved into the beam path. Obviously, the operation of controlling the actuator may be performed before actually emitting radiation by the source, such that the reference to the beam path refers to the expected beam path, as collimated by the collimator, when the source is activated.

The method comprises forming 13 a first image representative of radiation attenuation of an object or subject placed in the examination area by processing image signals provided by all the detector elements that were exposed to the radiation beam, and, if said selected ratio is non-zero, forming 14 at least a second image representative of phase contrast and/or small-angle scattering by processing image signals provided by only the detector elements that were exposed to the radiation beam and passing through the analyzer grating. The former and latter mentioned signals may be acquired in a single radiation exposure or scan operation. For forming the first image, a gain correction or a suitable processing step may be applied to compensate for a higher attenuation due to the presence of the analyzer grating for some of the detector elements.

The method may comprise moving 16, e.g. rotating, the radiation source and image detector, e.g. around a longitudinal axis, while acquiring said signals, e.g. to acquire a CT scan of the object or subject. The method may comprise moving 17 the object or subject along the longitudinal axis while rotating the source and detector around the longitudinal axis, e.g. performing a helical CT scan or a "step-and-shoot" CT scan.

The step of controlling 11 the collimator may comprise selecting an axial collimation that affects which detector rows of the image detector are exposed to the radiation beam. The detector rows may be arranged along the longitudinal axis with respect to one another. The analyzer grating may be arranged such that it extends over the entire angular arc that is subtended by the radiation detector, yet not the entire width of the radiation detector in the longitudinal direction, e.g. covers some detector rows entirely while leaving other detector rows entirely uncovered. Thus, the step of controlling the collimation may select a configurable number (possibly zero) of detector rows covered by the analyzer grating and a configurable number (possibly zero) of detector rows uncovered by the analyzer grating to be exposed by the radiation beam.

Forming the first image and second image may comprise performing a tomographic reconstruction of the signals.
In a fourth aspect, the present invention relates to a computer-program product for performing a method in accordance with embodiments of the third aspect of the present invention when executed on a computer.

## Claims

1. A grating-based X-ray imaging system (100) comprising:
- a radiation source (108) and a radiation image detector (110), wherein said radiation source and said radiation image detector are disposed at opposite sides of an examination region (106) such that, in use, radiation emitted by the radiation source passes through the examination region and impinges on the radiation image detector; and
- an X-ray interferometer for dark-field and/or phase-contrast imaging, the interferometer comprising an analyzer grating (101) arranged on or proximate to the radiation image detector (110),
wherein said analyzer grating only covers a part of the active area of the radiation image detector, such that a remainder (103) of the active area remains uncovered by the analyzer grating.

2. The imaging system of claim 1, wherein said analyzer grating (101) covers one side of the detector from an edge of the detector over only a part of the length of the detector along a first direction of the detector such that the remainder (103) of the active area of the detector on the other side, opposite of said edge in said first direction, remains uncovered by the analyzer grating.

3. The imaging system of claim 1, wherein said analyzer grating (101) covers both sides of the detector, at opposite ends of the detector in a first direction, while leaving a central part uncovered.

4. The imaging system of claim 1 or claim 2, wherein said analyzer grating (101) covers at least 90% of the detector in a direction perpendicular to said first direction, and this over the entire length of the detector in said first direction.

5. The imaging system of any of the previous claims, wherein said x-ray interferometer comprises a source grating (105) and/or a third grating (107), the system further comprising an actuator mechanism for controllably moving the source grating and/or the third grating into and out of the beam path of radiation emitted by the radiation source to impinge on the radiation detector.

6. The imaging system of any of the previous claims, comprising an adjustable collimator (109) to block part of the radiation emitted by the radiation source such as to illuminate a selectable part of the detector by the unblocked portion of the beam emitted by the source.

7. The imaging system of claim 6, comprising a controller (111) for controlling the adjustable collimator (109) such as to select from different supported modes of operation, in which said different modes differ in the ratio of the detector area exposed to radiation emitted by the radiation source and covered by the analyzer grating and the detector area exposed to radiation emitted by the radiation source and uncovered by the analyzer grating.

8. The imaging system of any of the previous claims, comprising a rotatable gantry (104) and/or an actuatable arm onto which the radiation source (108) and the radiation image detector (110) are mounted at opposite positions with respect to each other across the examination region (106) such that the radiation source and the radiation image detector can be moved in a coordinated motion to acquire projection images of the examination region (106) from a plurality of different projection directions.

9. The imaging system of claim 8, wherein said imaging system is a computed tomography system, the imaging system comprising a stationary gantry (102) supporting the rotatable gantry (104); wherein the rotatable gantry is adapted for rotating around the examination region (106) around a longitudinal axis (Z).

10. The imaging system of claim 9, comprising a reconstructor (112) for reconstructing tomographic images from signals provided by the image detector (110), wherein said reconstructor is adapted for performing a phase contrast and/or dark-field image reconstruction based on those of said signals that were obtained by elements of the image detector covered by the analyzer grating that were exposed to radiation emitted by the radiation source and for performing an attenuation image reconstruction based on all of said signals that were obtained by elements of the image detector that were exposed to radiation emitted by the radiation source.

11. The imaging system of claim 9 or claim 10, comprising a support (113) for supporting an object or subject to be imaged in the examination region (106), wherein said support comprises one or more actuators to move the support, and the subject or object thereon, through the examination region such as to scan the subject or object over a length in the longitudinal direction (Z).

12. The imaging system of any of the claims 9 to 11, wherein said analyzer grating (101) is oriented such that it entirely covers the angular arc that is subtended by the radiation detector, yet not the entire width of the radiation detector in the longitudinal direction (Z).

13. An interferometer grating assembly (1) comprising a mounting frame encompassing an area, wherein said analyzer grating covers a fraction of said area in the range of 10% to 90%, and wherein a remainder (103) of said fraction of the area is left open or filled with a material that does not significantly attenuate X-ray radiation.

14. A method (10) for operating a grating-based X-ray imaging system that comprises a radiation source and a radiation image detector, disposed at opposite sides of an examination region, and an X-ray interferometer for dark-field and/or phase-contrast imaging,
wherein said interferometer comprises an analyzer grating arranged on or proximate to the radiation image detector,
wherein said analyzer grating only covers a part of the active area of the radiation image detector, such that a remainder of the active area remains uncovered by the analyzer grating,
the method comprising:
- controlling (11) a collimator of the system for collimating a radiation beam emitted by the radiation source such as to configure a selectable ratio of the detector area exposed to the radiation beam for which the radiation passes through the analyzer grating relative to the detector area exposed to the radiation beam for which the radiation does not pass through the analyzer grating,
- emitting (12) said radiation beam by the radiation source and through the examination region to impinge on the radiation image detector,
- forming (13) a first image representative of radiation attenuation of an object or subject placed in the examination area by processing image signals provided by all of the detector elements of said radiation image detector that were exposed to the radiation beam, and, if said selected ratio is non-zero, forming (14) at least a second image representative of phase contrast and/or small-angle scattering by processing image signals provided by only the detector elements that were exposed to the radiation beam where said beam passed through the analyzer grating.

15. A computer-program product for performing a method in accordance claim 14 when executed on a computer.
